# EUROPEAN PATENT SPECIFICATION

(11) **EP 3 146 978 B1**
(45) Date of publication and mention of the grant of the patent: **20.07.2022**
(21) Application number: 15796913.0
(22) Date of filing: 20.05.2015
(51) Int. Cl.: A61K 31/198, A61K 45/00, A61K 31/192, A61K 31/196, A61P 35/00, A61P 43/00, C12N 5/095

(54) **INHIBITION OF CANCER STEM CELL PROLIFERATION**
INHIBITION DER KREBSSTAMMZELLENPROLIFERATION
INHIBITION DE LA PROLIFÉRATION DES CELLULES SOUCHES CANCÉREUSES

(30) Priority: 21.05.2014 JP 2014105543
(43) Date of publication of application: 29.03.2017
(73) Proprietor: National Institute of Advanced Industrial Science and Technology, Tokyo 100-8921 (JP); TMRC Co.,Ltd., Shinjuku-ku Tokyo 1690074 (JP)
(72) Inventor: KURISAKI Akira, Tsukuba-shi Ibaraki 305-8561 (JP); WANG Ying Ying, Tsukuba-shi Ibaraki 305-8561 (JP); TAKADA Hitomi, Tsukuba-shi Ibaraki 305-8561 (JP); EKIMOTO Hisao, Tokyo 115-0042 (JP)
(74) Representative: Wallace, Sheila Jane
(86) International application number: PCT/JP2015/064523
(87) International publication number: WO 2015/178426

(56) References cited:
- EP-A2- 2 005 954
- WO-A1-2007/108272
- US-A1- 2009 227 674
- MARCIA I DAWSON ET AL: "The retinoid X receptors and their ligands", BIOCHIMICA ET BIOPHYSICA ACTA (BBA) - MOLECULAR AND CELL BIOLOGY OF LIPIDS, ELSEVIER, AMSTERDAM, NL, vol. 1821, no. 1, 23 September 2011 (2011-09-23), pages 21-56, XP028391787, ISSN: 1388-1981, DOI: 10.1016/J.BBALIP.2011.09.014 [retrieved on 2011-10-01]
- DATABASE MEDLINE [Online] US NATIONAL LIBRARY OF MEDICINE (NLM), BETHESDA, MD, US; June 2007 (2007-06), USUBA TERUYUKI ET AL: "Combination chemotherapy using TS-1, Paclitaxel and cisplatin for multiple lung metastases from AFP-producing gastric cancer: a case report.", XP002775162, Database accession no. NLM17629094 & USUBA TERUYUKI ET AL: "Combination chemotherapy using TS-1, Paclitaxel and cisplatin for multiple lung metastases from AFP-producing gastric cancer: a case report.", HEPATO-GASTROENTEROLOGY JUN 2007, vol. 54, no. 76, June 2007 (2007-06), pages 1302-1304, ISSN: 0172-6390
- GINESTIER, C. ET AL.: 'Retinoid signaling regulates breast cancer stem cell differentiation' CELL CYCLE vol. 8, no. 20, 2009, pages 3297 - 302, XP055001900
- LIM, Y. C. ET AL.: 'All-trans-retinoic acid inhibits growth of head and neck cancer stem cells by suppression of Wnt/beta-catenin pathway' EUR. J. CANCER. vol. 48, no. 17, 2012, pages 3310 - 8, XP055164476
- WHITWORTH, J. M. ET AL.: 'The impact of novel retinoids in combination with platinum chemotherapy on ovarian cancer stem cells' GYNECOL. ONCOL. vol. 125, no. 1, 2012, pages 226 - 230, XP055238566
- OHTA, K. ET AL.: 'Potent retinoid synergists with a diphenylamine skeleton' BIOL PHARM BULL vol. 21, no. 5, 1998, pages 544 - 6, XP055238568
- SHUICHI OKADA: 'Atarashii Koganzai no Tenkai' SHOKAKIBYO SEMINAR vol. 80, 2000, pages 167 - 75, XP008185811
- OKAMURA, T. ET AL.: 'Activation of Retinoic Acid Receptor a is Important for the Anti-myeloma Effect of Retinoids: New Therapeutic Approach Using Receptor Selective Retinoids for Multiple Myeloma' JOURNAL OF TOKYO WOMENS MEDICAL UNIVERSITY vol. 83, 2013, pages E576 - E584, XP008185812
- NALLS, D. ET AL.: 'Targeting epigenetic regulation of miR-34a for treatment of pancreatic cancer by inhibition of pancreatic cancer stem cells' PLOS ONE vol. 6, no. 8, 2011, pages 1 - 12, XP055360806 Retrieved from the Internet: <URL:http://journals.plos.org/plosone/artic le ? id=10.1371/journal.pone.0024099> [retrieved on 2015-06-22]
- I. Miwako ET AL: "Tamibarotene", DRUGS OF TODAY, vol. 43, no. 8, 1 January 2007 (2007-01-01), page 563, XP055419885, ES ISSN: 1699-3993, DOI: 10.1358/dot.2007.43.8.1072615
- Adis International Limited: "Tamibarotene: AM 80, Retinobenzoic Acid, Tamibaro", Drugs in R&D, 1 January 2004 (2004-01-01), pages 359-362, XP055742410, Cham DOI: 10.2165/00126839-200405060-00010 Retrieved from the Internet: URL:https://link.springer.com/article/10.2 165/00126839-200405060-00010 [retrieved on 2020-10-21]

## Description

### TECHNICAL FIELD

The present invention relates to a composition comprising tamibarotene (Am-80) and a further anticancer drug for use in reducing cancer stem cells in a subject. The invention is solely defined by the appended claims.

### BACKGROUND ART

Among the advances in medicine, treatment of cancer is an area making an extremely rapid progress, and a variety of therapeutic methods for cancer are available. Nevertheless, based on surveys of vital statistics by the Japanese Ministry of Health, Labour and Welfare, and surveys by the World Health Organization, the number of deaths due to cancer is expected to increase also in the future. As one of its causes, the existence of cancer stem cells, which show resistance to conventional anticancer drug therapies, is drawing attention.

Cancer cells gain high growth capacity, infiltration capacity, and metastatic capacity due to accumulation of genetic mutations, small environmental changes in the vicinity of the cancer cells, and the like. However, not all of the cancer cells constituting a cancer mass have these characteristics. Recently, it has become clear that only very small proportions of cancer cells have these characteristics, and therefore have high capacity to generate, and allow the progression of cancer (Non-patent Document 1). Cancer cells having such characteristics are called cancer stem cells since these cells have two characteristics commonly found in stem cells, the capacity of self-renewal, and pluripotency, which allows differentiation into many types of cells. The existence of cancer stem cells has been found in cancer species such as acute leukemia, breast cancer, colon cancer, brain tumor, prostate cancer, and pancreatic cancer.

Recently, it has become clear that cancer stem cells show resistance to conventional anticancer drug therapies. For example, in acute leukemia, cancer stem cells stay in a microenvironment called stem cell niche, and enter into the resting phase (Go phase), during which cell division does not occur. In this state, the cancer stem cells are resistant to anticancer drug therapies. Stem cells of solid cancers often show high expression of P-glycoprotein and the like, and show resistance to anticancer drugs. It is also known that the ratio of cancer stem cells present in a cancer tissue after an anticancer drug therapy is higher than that before the therapy.

These facts are thought to be major causes preventing complete cure of cancer after an anticancer drug therapy, leading to recurrence of the cancer. It has thus become clear that development of a therapeutic agent targeting cancer stem cells is an important method for complete cure of cancer. Therapeutic agents targeting cancer stem cells have so far been reported to suppress the growth of cancer and to enhance therapeutic effects of existing anticancer drugs. However, no therapeutic agent targeting cancer stem cells has been established at the clinical sites at present, and development of such a therapeutic agent is currently demanded (Non-patent Document 2). In view of this, the present inventors carried out a study using cancer stem cell markers in order to develop a novel, clinically applicable therapeutic agent targeting cancer stem cells (Non-patent Document 3). Reference is also made to EP 2005954; Biochimica et Biophysica Acta - Molecular and Cell Biology of Lipids, Vol. 1821, No. 1, 2011, pages 21-56; US 2009/227674; Hepato-Gastroenterology, Vol. 54, No. 76, 2007, pages 1302-1304; Cell Cycle, Vol. 8, No. 20, 2009, pages 3297-3302; Eur. J. Cancer, Vol 48, No. 17, 2012, pages 226-230; Biol. Pharm. Bull., Vol. 21, No. 5, 1998, pages 544-6; Journal of Tokyo Womens Medical University, Vol. 83, 2013, pages E576-E584; Plos One, Vol. 6, No. 8, 2011, pages 1-12; Drugs of Today, Vol. 43, No. 8, 2007, page 563 and Drugs in R&D, 2004, pages 359-362.

### RELATED ART DOCUMENTS

### NON PATENT DOCUMENTS

Non-patent Document 1: Proc. Natl. Acad. Sci. USA 100, 3983-3988 (2003)
Non-patent Document 2: Nature 453, 338-344 (2008)
Non-patent Document 3: Mol. Cancer Res. 7, 330-338 (2009)

### SUMMARY OF THE INVENTION

### PROBLEMS TO BE SOLVED BY THE INVENTION

However, growth inhibitors for cancer stem cells which can more effectively suppress the growth of cancer stem cells showing resistance to the existing anticancer drug therapies are still strongly demanded.

In view of this, the present invention aims to provide a composition comprising tamibarotene (Am-80) and a further anticancer drug for use in reducing cancer stem cells in a subj ect.

### MEANS FOR SOLVING THE PROBLEMS

In order to solve the above problems, the present inventors intensively studied focusing on signaling pathways in cancer stem cells.

That is, the undifferentiated state of cancer stem cells is maintained by many intracellular signaling pathways, and, by this, their ability to maintain cancer is retained. Substances that inhibit these signaling pathways can potentially be novel anticancer drugs that inhibit the growth of cancer by induction of differentiation or apoptosis of cancer stem cells.

In view of this, the present inventors used retinoids and rexinoids to study growth-inhibitory actions against cancer stem cells such as differentiation and apoptosis, among the physiological actions exerted by retinoids and rexinoids including growth, survival, differentiation, and apoptosis. During the course of the study, the present inventors considered that retinoids and rexinoids can be novel anticancer drugs.

Since the population of cancer stem cells constitutes only a part of cancer cells, cancer stem cell markers were used as indices for the study of growth-inhibitory actions on cancer stem cells. Since human pancreatic cancer cells express various cancer stem cell markers, those cells were used for the study.

As a result, it was found that the cell number, morphology, and expression of stem cell markers of human pancreatic cancer cells can be suppressed using a retinoid agonist, especially tamibarotene (Am-80) alone or in combination with a rexinoid agonist, especially bexarotene. Since retinoid receptors (RARs) and rexinoid receptors (RXRs) are intranuclear transcription factors, they may interact with compounds and agents that suppress epigenetic actions. In view of this, the present inventors used the retinoid agonist and/or rexinoid agonist in combination with an anticancer drug containing such an inhibitory compound or molecular-targeted agent having a different action mechanism, and discovered that the combination allows exertion of a drastic action to inhibit the growth of cancer stem cells. The present invention was completed based on such a discovery.

### EFFECTS OF THE INVENTION

In the present disclosure cancer stem cells can be reduced by administration of a retinoid agonist alone or in combination with a rexinoid agonist. By further administering an anticancer drug, the tumor size can be decreased or reduced. Simultaneous administration of a retinoid agonist and a rexinoid agonist together with an anticancer drug is also useful. Examples of cancers which may be treated by the present invention include blood cancers such as acute myeloid leukemia and non-Hodgkin's lymphoma; gastrointestinal cancers such as pancreatic cancer, hepatoma, and colon cancer; lung cancer; breast cancer; prostate cancer; and ovarian cancer; which show resistance to anticancer drug therapies.

### BRIEF DESCRIPTION OF THE DRAWINGS

Fig. 1 is a graph showing the changes in the cell number observed after 1 week of suspension culture of Panc-1 in the presence of Am80 at different concentrations.
Fig. 2 shows graphs showing the decreases in the number of ALDH-positive cells observed after treatment of Panc-1 with Am80 under suspension culture conditions.
Fig. 3 shows graphs showing the decreases in the number of CD24/CD44/ESA-positive cells observed after treatment of Panc-1 with Am80 under suspension culture conditions.
Fig. 4 shows graphs showing the increases in the levels of pancreatic tissue differentiation markers observed after culturing Panc-1 in the presence of Am80 under suspension culture conditions.
Fig. 5 is a graph showing suppression of the cell growth activity by 1 week of co-treatment of MIA Paca2 cells with Am80 and bexarotene.
Fig. 6 is a graph showing suppression of the cell growth activity by 1 week of co-treatment of MIA Paca2 cells with Am80 and 5-aza-dC.
Fig. 7 is a graph showing suppression of the cell growth activity by 1 week of co-treatment of MIA Paca2 cells with Am80 and SAHA.
Fig. 8 is a graph showing suppression of the cell growth activity by 1 week of co-treatment of MIA Paca2 cells with Am80 and VPA.
Fig. 9 is a micrograph showing the influence of Am80 on the cell migration ability of human pancreatic cancer cells.
Fig. 10 is a graph showing the anti-metastatic effect of Am80 on human pancreatic cancer cells.
Fig. 11 is a graph showing the anti-tumorigenic effect of Am80 on human pancreatic cancer cells.
Fig. 12 is a graph showing the inhibitory effect of Am80 on human pancreatic cancer stem cells transplanted to nude mice.
Fig. 13 is a photograph of pathologic sample showing the anti-tumorigenic effect of Am80 on human pancreatic cancer cells.
Fig. 14 is a graph showing the *in vitro* combined effect of Am80 and an anticancer drug gemcitabine against human pancreatic cancer cells.
Fig. 15 is a graph showing the *in vivo* combined effect of Am80 and an anticancer drug gemcitabine against human pancreatic cancer cells/nude mice.
Fig. 16 is a graph showing the *in vivo* combined effect of Am80 and an epigenetic-action inhibitor VPA against human pancreatic cancer cells/nude mice.
Fig. 17 is an isobologram showing the combined effect of Am80 and 5-AZ against human leukemia cells Kasumi-1.
Fig. 18 is a graph showing suppression of the cell growth activity by 96 hours of co-treatment of MCF-7 cells with Am80 and 5-AZ.
Fig. 19 is a graph showing suppression of the cell growth activity by 96 hours of co-treatment of LNCaP cells with Am80 and 5-AZ.

### MODE FOR CARRYING OUT THE INVENTION

Modes for carrying out the present invention are described below concretely.

The growth inhibitor for cancer stem cells of the present invention comprises a retinoid agonist as an effective component. In the present invention, the retinoid agonist includes its pharmaceutically acceptable organic or inorganic acid addition salts. The retinoid agonist used in the present invention is tamibarotene (Am80).

The growth inhibitor for cancer stem cells of the present disclosure can be preferably used in combination with a rexinoid agonist. The rexinoid agonist also includes its pharmaceutically acceptable organic or inorganic acid addition salts, and example of especially preferred rexinoid agonists includes bexarotene (Targretin).

When used in combination with an anticancer drug, the growth inhibitor for cancer stem cells of the present disclosure has an action to enhance the anticancer activity of the anticancer drug.

In the present invention, examples of the DNA-interacting agents which may be used as the anticancer drug include alkylating agents such as cyclophosphamide, melphalan, cisplatin, and carboplatin; DNA topoisomerase I inhibitors such as camptothecin; and DNA topoisomerase II inhibitors such as etoposide, daunorubicin, and doxorubicin. Preferred examples of the DNA-interacting agents include cyclophosphamide, melphalan, cisplatin, camptothecin, and doxorubicin, which can be orally administered.

Examples of the antimetabolites which may be used as the anticancer drug include folate antagonists such as methotrexate and trimethotrexate; pyrimidine antagonists such as 5-fluorouracil, 5-aza-dC, azacytidine, cytarabine, and gemcitabine; and purine antagonists such as fludarabine. Preferred examples of the antimetabolites include methotrexate, 5-fluorouracil, 5-aza-dC, azacytidine, cytarabine, gemcitabine, and fludarabine, which can be orally administered.

Preferred examples of the tubulin-interacting agents which may be used as the anticancer drug include paclitaxel and docetaxel.

Examples of the molecular-targeted therapeutic agents which may be used as the anticancer drug include cycloxygenase 2 inhibitors such as celecoxib and rofecoxib; and growth factor signaling inhibitors such as erlotinib and imatinib. Preferred examples of the molecular-targeted therapeutic agents include gefitinib and imatinib, which can be orally administered.

Examples of the epigenetic-action inhibitors which may be used as the anticancer drug include DNA methylation inhibitors such as azacytidine; and histone deacetylase inhibitors such as valproic acid, SAHA (Vorinostat), and Romidepsin (FK228). Preferred examples of the epigenetic-action inhibitors include azacytidine, valproic acid, and SAHA (Vorinostat), which can be orally administered.

Examples of the hormones which may be used as the anticancer drug include leuprolide acetate; goserelin acetate; antiestrogens such as tamoxifen; and antiandrogens such as flutamide, bicalutamide, and enzalutamide. Preferred examples of the hormones include tamoxifen, flutamide, bicalutamide, and enzalutamide, which can be orally administered.

Examples of combinations of the anticancer drugs include, but are not limited to, the combination of paclitaxel (or docetaxel), cisplatin, and TS-1; combination of docetaxel and oxaliplatin; and combination of docetaxel and a molecular-targeted agent.

The growth inhibitor for cancer stem cells of the present invention can effectively inhibit the growth of cancer stem cells and cancer masses in human when an effective amount of the growth inhibitor is administered orally, rectally, topically, or parenterally, or by intravenous injection or intratumoral injection.

The growth inhibitor for cancer stem cells of the present disclosure may be in the form of a solid such as a tablet, capsule, ball, or liposome, in the form of a gel, or in the form of a liquid (see Cancer Chemotherapy Handbook version 2, 15-34 (1994)).

In therapies using the growth inhibitor for cancer stem cells of the present invention, the growth inhibitor may be used at any dose as long as the dose is appropriate for the type of the cancer to be treated. The method of applying the effective dose varies depending on the abundance of the cancer stem cells as the subject of the treatment. For example, a total of 0.5 to 500 mg per human individual of the retinoid agonist and the rexinoid agonist, and 1.0 to 1000 mg per human individual of the anticancer drug, may be contained. The retinoid agonist may be preferably contained in an amount of 0.5 to 20 mg per human individual.

As the growth inhibitor for cancer stem cells of the present disclosure, the retinoid agonist alone, or in combination with the rexinoid agonist, is administered together with the anticancer drug to cause differentiation of cancer stem cells, which leads to apoptosis and then growth inhibition or reduction of the cancer cells. The growth inhibitor may be administered for 2 weeks to 2 years as required in order to suppress the growth of cancer stem cells.

### EXAMPLES

The present invention is described below by way of Examples. However, the following Examples merely describe exemplary cases, and, of course, do not limit the present invention.

### [Reference Example 1]

### <Growth Inhibitory Effect of Am80 on Human Pancreatic Cancer Cell Spheroids>

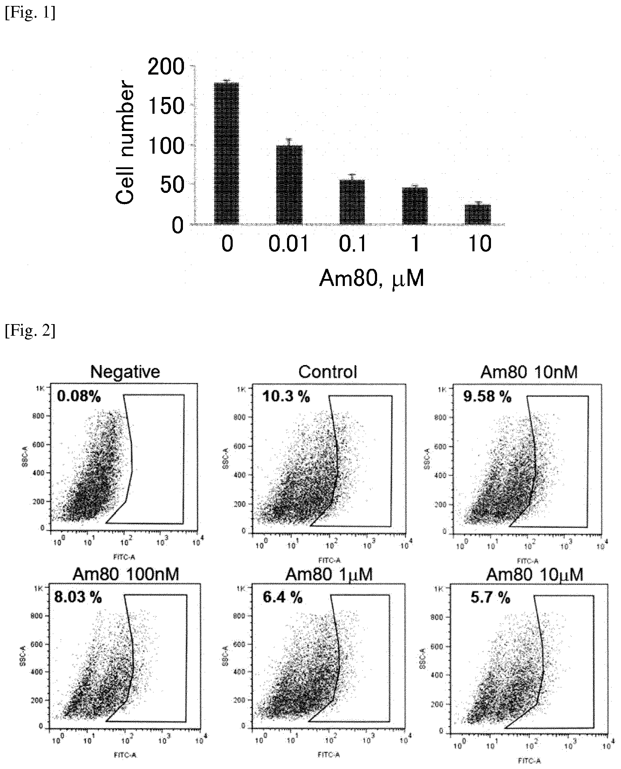

Culturing of a human pancreatic cancer cell line in DMEM/F12 medium supplemented with B27, 20 ng/mL EGF, 20 ng/mL bFGF, and 4 µg/mL heparin using a low-adhesion dish allows formation of cell clusters, and the cells can be cultured in a suspended state. Under these conditions, the cells can be cultured such that they show increased levels of several cancer stem cell markers as well as markers reported for other cancers. First, the cell clusters containing the cells positive for the cancer stem cell markers were cultured in a medium supplemented with Am80 for 1 week, and the influences of Am80 on the growth of cell clusters and the expression levels of the cancer stem cell markers were analyzed. As a result, the growth of the cell clusters was suppressed dependently on the concentration of tamibarotene (Am80). The number of cell clusters decreased, and the number of cells also decreased dependently on the concentration (Fig. 1). When the cell clusters were further cultured for a total of 2 weeks in the Am80-containing medium, the sizes of the cell clusters further decreased, and the number of cells decreased. From these results, Am80 was found to have an action to inhibit the growth of a cell population containing pancreatic cancer stem cells.

### [Reference Example 2]

### <Inhibitory Effect of Am80 on ALDH Activity of Human Pancreatic Cancer Cell Panc-1 Spheroids>

As shown in Example 1, Am80 suppresses formation of cell clusters of the pancreatic cancer cell line in the suspension-culture state, and this might be due to a decrease in the number of the stem cells that act as the seeds of the cell clusters. It is known that the activity of aldehyde dehydrogenase (ALDH) is high in hematopoietic stem cells and progenitor cells, but low in differentiated cells. In view of this, a precursor Bodipy-aminoacetaldehyde (ALDEFLUOR, Stem Cell Technologies) was added to pancreatic cancer cells prepared by performing suspension culture in the presence of Am80 for 1 week, and the number of cells positive for the fluorescence of Bodipy-aminoacetate produced by metabolism by the aldehyde dehydrogenase activity was analyzed by quantification by flow cytometry. As a result, it was shown that there is a possibility that culturing in the presence of Am80 causes a concentration-dependent decrease in the ALDH activity, resulting in a decrease in the amount of cancer stem cells (Fig. 2).

### [Reference Example 3]

### <Inhibitory Effect of Am80 on CD24+/CD44+/ESA+ Cells Present in Human Pancreatic Cancer Cell Panc-1 Spheroids>

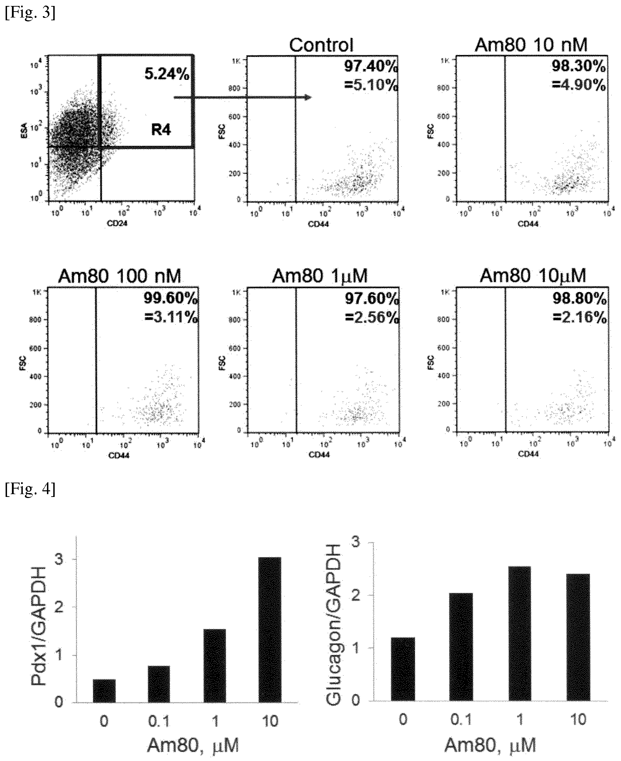

Pancreatic cancer cells prepared by performing suspension culture in the presence of Am80 for 1 week was subjected to analysis of expression of cancer stem cell surface markers at the protein level by flow cytometry using fluorescence-labeled primary antibodies. As a result, in particular, it was found that the abundance of cells positive for CD24+/CD44+/ESA+ was significantly decreased by the addition of Am80. In particular, the abundance of cells positive for CD24+/CD44+/ESA+ was reduced by half in the presence of 10 µM Am80. That is, it was suggested that Am80 might decrease cancer stem cells (Fig. 3).

### [Reference Example 4]

### <Effect to Enhance Differentiation Markers in Human Pancreatic Cancer Cell Spheroids>

Subsequently, total RNA was extracted from pancreatic cancer cells prepared by performing suspension culture in the presence of Am80 for 1 week, and quantitative RT-PCR was carried out using primers specific to pancreatic tissue-specific differentiation markers. As a result, Pdx1, glucagon, and the like were found to show increased gene expression dependently on the Am80 concentration. Thus, it was suggested that Am80 might promote differentiation of cell populations containing cancer stem cells (Fig. 4).

### [Reference Example 5]

### <Influence of Combination of Am80 and Bexarotene on Cell Growth Activity of Human Pancreatic Cancer Cell MIA-Paca2 Spheroids>

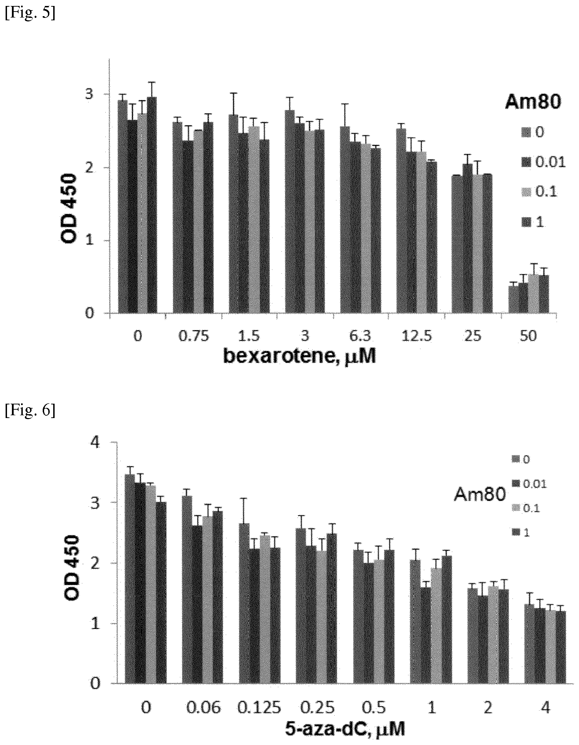

MIA-Paca2 cell clusters containing cells positive for cancer stem cell markers were subjected to suspension culture in a medium supplemented with Am80 (10 nM-1 µM) and bexarotene for 1 week, and 3 hours of additional culture was carried out after adding an MTT reagent (Cell Counting Kit-8, Dojindo), followed by analyzing the influence of the agents on the cell growth activity of the cell clusters by measurement of the absorbance at 450 nm (OD 450). As a result, it was found that the cell growth activity of the cell clusters was slowly suppressed dependently on the concentrations of Am80 and bexarotene (Fig. 5).

### [Example 6]

### <Influence of Combination of Am80 and DNA Methylation Inhibitor 5-aza-dC on Growth Activity of Human Pancreatic Cancer Cell MIA-Paca2 Spheroids>

MIA-Paca2 cell clusters containing cells positive for cancer stem cell markers were subjected to suspension culture in a medium supplemented with Am80 (10 nM-1 µM) and 5-aza-dC for 1 week, and 3 hours of additional culture was carried out after adding an MTT reagent (Cell Counting Kit-8, Dojindo), followed by analyzing the influence of the agents on the cell growth of the cell clusters by measurement of the absorbance at 450 nm. As a result, it was found that the cell growth activity of the cell clusters was suppressed dependently on the concentrations of Am80 and 5-aza-dC (Fig. 6).

### [Example 7]

### <Influence of Combination of Am80 and Histone Deacetylase Inhibitor SAHA on Growth Inhibition of Human Pancreatic Cancer Cell MIA-Paca2 Spheroids>

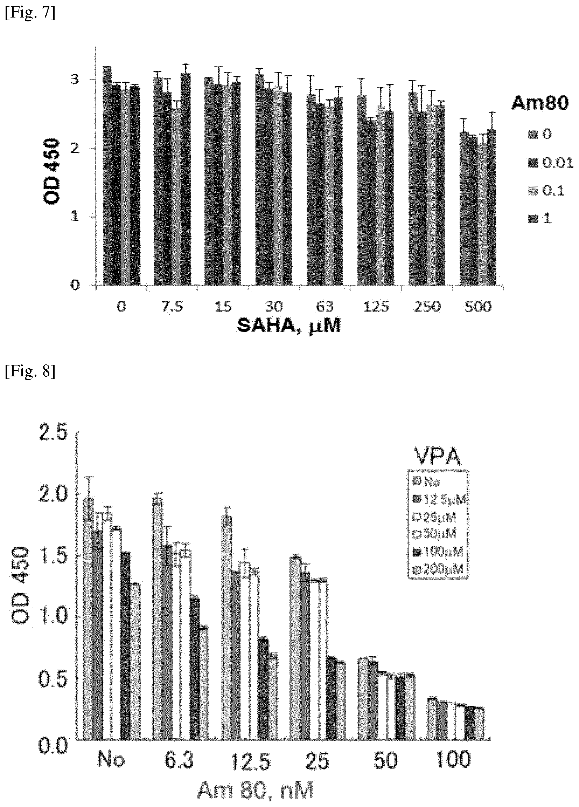

MIA-Paca2 cell clusters containing cells positive for cancer stem cell markers were subjected to suspension culture in a medium supplemented with Am80 (10 nM-1 µM) and SAHA for 1 week, and 3 hours of additional culture was carried out after adding an MTT reagent (Cell Counting Kit-8, Dojindo), followed by analyzing the influence of the agents on the cell growth activity of the cell clusters by measurement of the absorbance at 450 nm. As a result, it was found that the cell growth activity of the cell clusters was slowly suppressed dependently on the concentrations of Am80 and SAHA (Fig. 7).

### [Example 8]

### <Influence of Combination of Am80 and Histone Deacetylase Inhibitor Valproic Acid (VPA) on Growth Inhibition of Human Pancreatic Cancer Cell MIA-Paca2 Spheroids>

MIA-Paca2 cell clusters containing cells positive for cancer stem cell markers were subjected to suspension culture in a medium supplemented with Am80 (6.3 nM-100 nM) and VPA for 1 week, and 3 hours of additional culture was carried out after adding an MTT reagent (Cell Counting Kit-8, Dojindo), followed by analyzing the influence of the agents on the cell growth of the cell clusters by measurement of the absorbance at 450 nm. As a result, it was found that Am80 and VPA showed a synergistic inhibitory activity on the cell growth of the cell clusters (Fig. 8).

### [Reference Example 9]

### <Influence of Am80 on Cell Migration Ability of Human Pancreatic Cancer Stem Cells>

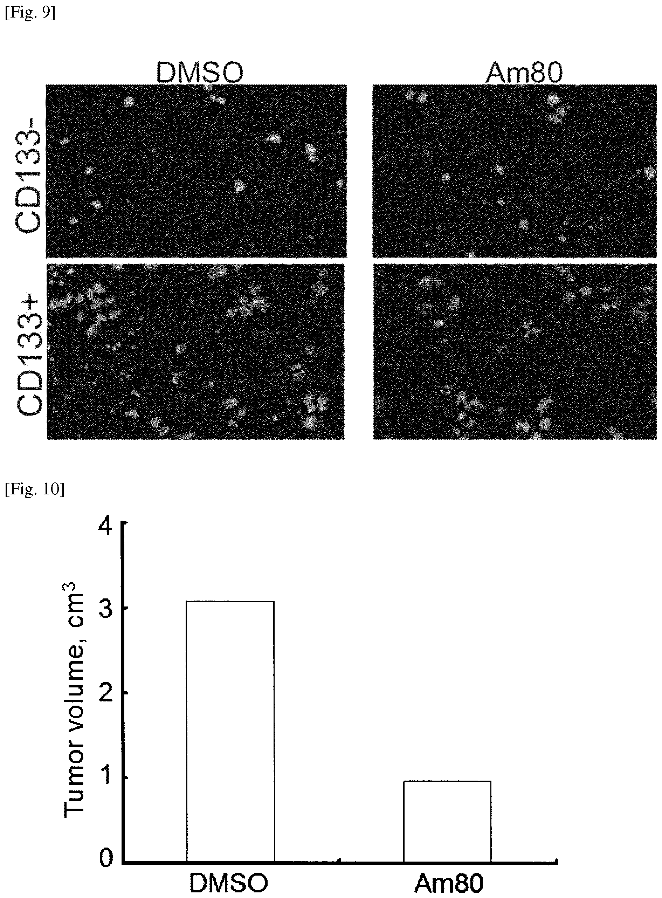

Pancreatic cancer cells containing pancreatic cancer stem cells were separated into CD133-positive cells and negative cells using magnetic antibody beads, and Am80 was added at 1 µM to the fractions, followed by culturing the cells in Boyden chambers and then observing the cells migrated into the back side of the membrane. As a result, Am80 was found to show remarkable suppression of the cell migration ability for the cancer stem cell fraction composed of the CD133-positive cells (Fig. 9).

### [Reference Example 10]

### <Anti-metastatic Effect of Am80 on Human Pancreatic Cancer Cells>

Pancreatic cancer cells (1 × 10⁶ cells) containing pancreatic cancer stem cells were transplanted to the pancreas of nude mice, and the size of a tumor formed by metastasis to the liver was measured 1 month later. Am80 was administered at 3 mg/kg/day for 1 month. As a result, metastasis of the pancreatic cancer could be suppressed compared to the control (Fig. 10).

### [Reference Example 11]

### <Anti-tumorigenic Effect of Am80 on Human Pancreatic Cancer Cells>

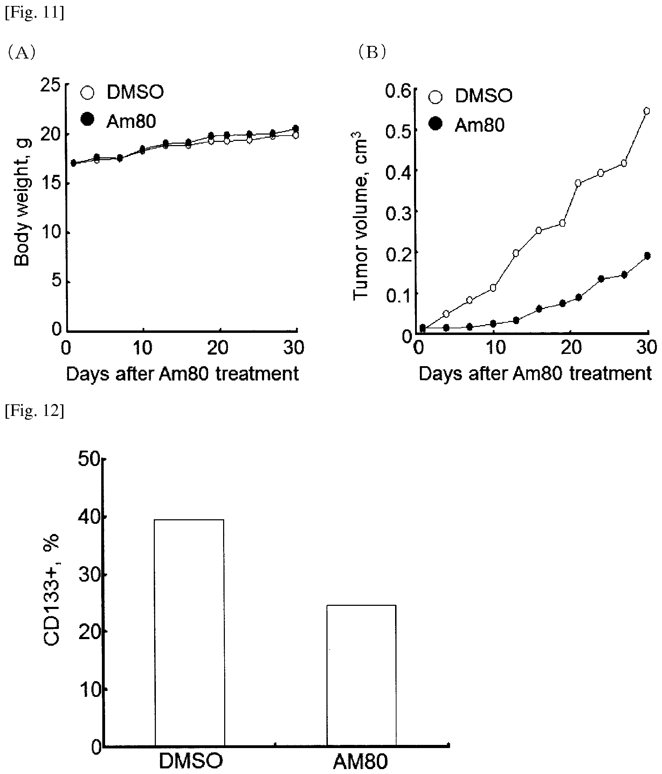

Pancreatic cancer cells (1 × 10⁶ cells) containing pancreatic cancer stem cells were subcutaneously transplanted to nude mice. Mice in which tumor formation was found 2 weeks later were subjected to administration of Am80 for 1 month at 3mg/kg/day. As a result, the body weight was not influenced by Am80 (A), but the growth of the pancreatic cancer could be suppressed *in vivo* compared to the control (B) (Fig. 11).

### [Reference Example 12]

### <Inhibitory Effect of Am80 on Human Pancreatic Cancer Stem Cells Transplanted to Nude Mice>

Pancreatic cancer cells (1 × 10⁶ cells) containing pancreatic cancer stem cells were subcutaneously transplanted to nude mice. Mice in which tumor formation was found 2 weeks later were subjected to administration of Am80 for 1 month at 3mg/kg/day. Thereafter, the tumor was removed, and the tumor cells were treated by trypsin, followed by measuring the ratio of pancreatic cancer stem cells by flow cytometry using an anti-CD133 antibody. As a result, it was found, based on comparison with the control, that Am80 has an action to suppress pancreatic cancer stem cells *in vivo* (Fig. 12).

### [Reference Example 13]

### <Anti-tumorigenic Effect of Am80 on Human Pancreatic Cancer Cells>

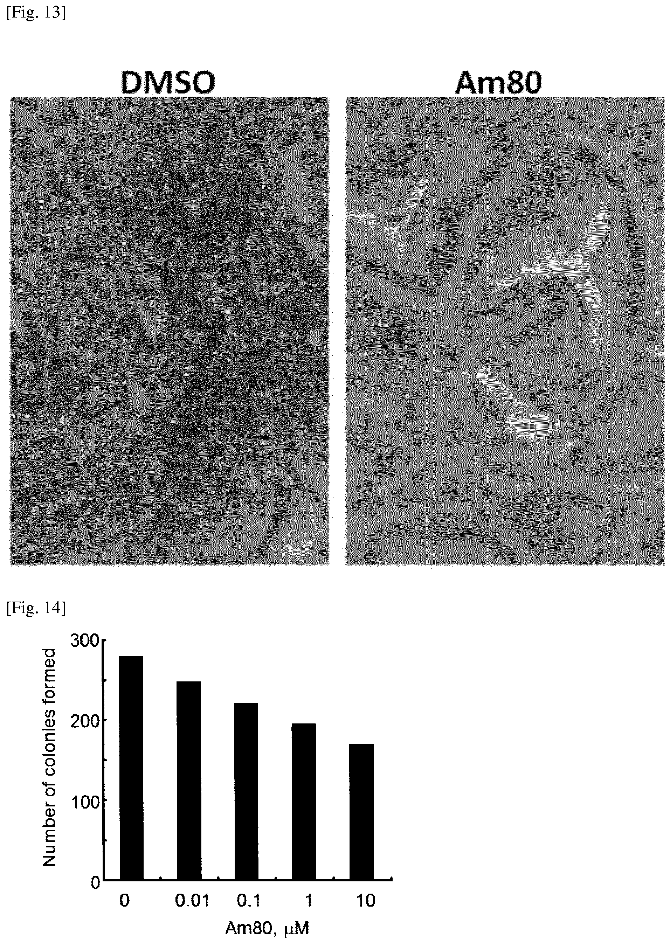

Pancreatic cancer cells (1 × 10⁶ cells) containing pancreatic cancer stem cells were subcutaneously transplanted to nude mice. Mice in which tumor formation was found 2 weeks later were subjected to administration of Am80 for 1 month at 3mg/kg/day. Pathologic samples of each tumor were prepared to evaluate morphology of cellular tissue. As a result, it was found, based on comparison with control group which is not administrated Am80, the pancreatic cancer tissue shows differentiated morphology (Fig. 13).

### [Example 14]

### <Effect of Combination of Anticancer Drug Gemcitabine and Am80 against Human Pancreatic Cancer Cells>

Human pancreatic cancer cells were cultured in the suspension-culture state in the presence of gemcitabine (10 nM) for 1 week. Thereafter, Am80 was added to the culture, and suspension culture was carried out for 1 week, followed by counting the number of colonies formed. As a result, it was found that Am80 also has an *in vitro* inhibitory effect on sphere formation of pancreatic cancer cells treated with an anticancer drug (Fig. 14).

### [Example 15]

### <Effect of Combination of Anticancer Drug Gemcitabine and Am80 against Human Pancreatic Cancer Cells>

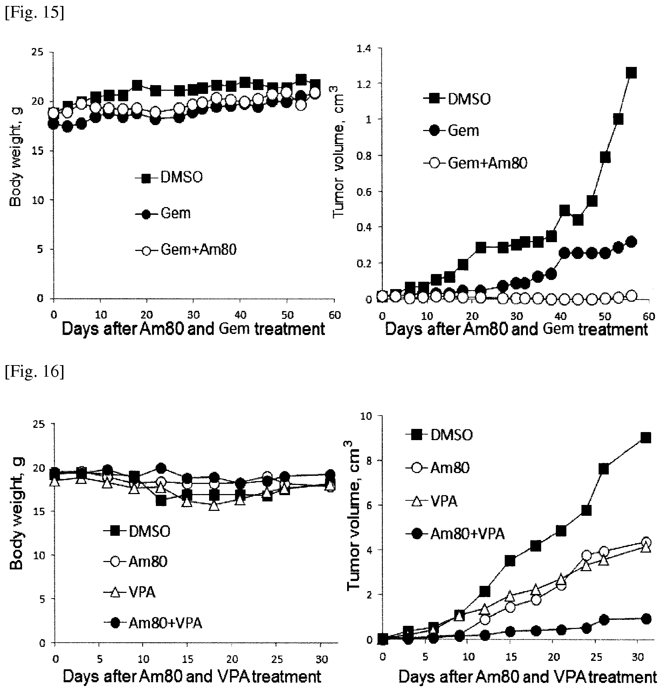

Pancreatic cancer cells (1 × 10⁶ cells) containing pancreatic cancer stem cells were subcutaneously transplanted to nude mice. Mice in which tumor formation was found 2 weeks later were subjected to daily administration of Am80 at 3mg/kg/day and administration of gemcitabine at 50 mg/kg at intervals of 3 days for 1 month. As a result, it was found that simultaneous administration of Am80 and gemcitabine more strongly suppresses the growth of pancreatic cancer *in vivo* compared to the control to which Am80 and gemcitabine were not administered and administration of gemcitabine alone (Fig. 15). Gem herein represents gemcitabine.

### [Example 16]

### <Effect of Combination of Epigenetic-action Inhibitor VPA and Am80 against Human Pancreatic Cancer Cells>

Pancreatic cancer cells (1 × 10⁶ cells) containing pancreatic cancer stem cells were subcutaneously transplanted to nude mice. Mice in which tumor formation was found 2 weeks later were subjected to daily administration of Am80 at 3mg/kg/day and administration of VPA at 500 mg/kg at intervals of 3 days for 1 month. As a result, it was found that simultaneous administration of Am80 and VPA more strongly suppresses the growth of pancreatic cancer *in vivo* compared to the control to which Am80 and VPA were not administered, administration of VPA alone and administration of Am80 alone (Fig. 16).

### [Example 17]

### <Influence of Combination of Am80 and Docetaxel (DXL) on Growth Inhibition of Human Pancreatic Cancer Cell MIA-Paca2/Nude Mice>

A tumor piece of human pancreatic cancer cells MIA-Paca2 containing cells positive for cancer stem cell markers was subcutaneously transplanted to the dorsal part of each of 6-week-old female BALB/cAJcl-nu nude mice (4 mice/group, 5 mice only for the control group) using a trocar needle. At the time when the tumor volume reached 100 to 200 mm³, the administration was started. Am80 was orally administered once a day at a dose of 2 mg/kg for 21 continuous days, and DXL was intravenously administered at a dose of 5 mg/kg from Day 1, 3 times at intervals of 4 days. In the combination group, the agents were administered at the same doses as, and by the same administration methods as, those in the cases of administration of each single agent. Measurement of the tumor diameter was carried out until the next day of the completion of the administration (Day 22). Based on the measured values of the longitudinal diameter and the transverse diameter, the tumor volume (mm³) was determined according to the following formula: longitudinal diameter × transverse diameter²/2. In the control group, physiological saline was intraperitoneally administered from Day 1, 3 times at intervals of 4 days. As a result, the combination of Am80 and DXL showed a synergistic inhibitory activity on the cell growth, which inhibitory activity was higher than the effect of each single agent. These results are shown in following Table 1.

**[Table 1]**

| | Day 1 | Day 5 | Day 9 | Day 13 | Day 17 | Day 22 |
|---|---|---|---|---|---|---|
| Control group | 140.8 | 222.9 | 300.3 | 453.7 | 648.7 | 862.5 |
| Am80 group | 151.3 | 182.1 | 255.5 | 348.8 | 429.9 | 466.4 |
| DXL group | 136.7 | 151.3 | 135.7 | 93.7 | 76.3 | 77.3 |
| Combination group | 138.2 | 131.6 | 110.3 | 71.2 | 60.1 | 41.2 |

### [Example 18]

### <Influence of Combination of Am80 and DNA Methylation Inhibitor Azacytidine (5-AZ) on Growth Inhibition of Human Leukemia Cell Line Kasumi-1>

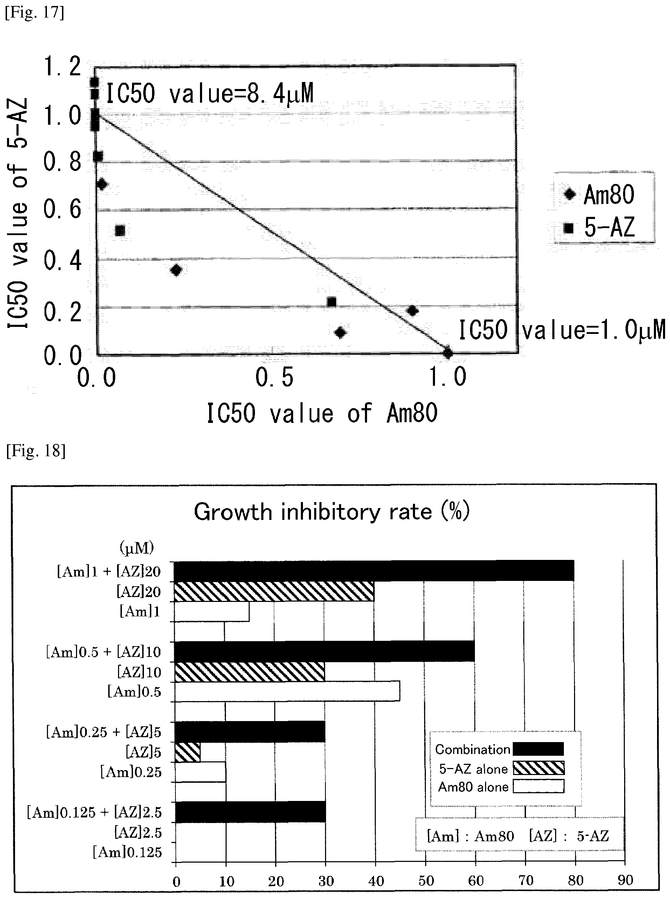

A human leukemia cell line Kasummi-1, containing cells positive for cancer stem cell markers, was cultured in RPMI1640 medium supplemented with 20% FBS, and the growth inhibitory action by the combination of Am80 and 5-AZ was studied using Isobologram. The IC50 values of the agents were 1.0 µM and 8.4 µM, respectively. These concentrations were taken as 1.0, and the points corresponding to 1.0 in the two axes were connected to each other via a straight line. In cases where the ratio observed for the combination effect is on the line, the effect is additive, and, in cases where the ratio is below the line, the effect is synergistic. The combination of Am80 and 5-AZ showed a synergistic effect in the growth inhibitory action (Fig. 17).

### [Example 19]

### <Influence of Combination of Am80 and DNA Methylation Inhibitor 5-AZ on Growth Inhibition of Human Breast Cancer Cell Line MCF-7>

A human breast cancer cell line MCF-7, containing cells positive for cancer stem cell markers, was cultured in RPMI1640 medium supplemented with 2% FBS, and the growth inhibitory action by the combination of Am80 and 5-AZ was studied. The IC50 values of the agents determined by 96 hours of the exposure were 1 µM and 20 µM, respectively. Thus, in order to study the growth inhibitory action, Am80 was used within the concentration range of 0.125 µM to 1 µM with a common ratio of 2, and 5-AZ was used within the concentration range of 2.5 µM to 20 µM with a common ratio of 2. In cases of use of these agents in combination, the growth inhibitory action was studied using these concentrations. The combination of Am80 and 5-AZ showed a synergistic effect on the growth inhibitory action (Fig. 18).

### [Example 20]

### <Influence of Combination of Am80 and Docetaxel (DXL) on Growth Inhibition of Human Breast Cancer Cell Line MCF-7/Nude Mice>

A tumor piece of a human breast cancer cell line MCF-7 containing cells positive for cancer stem cell markers was subcutaneously transplanted to the dorsal part of each of 6-week-old female BALB/cAJcl-nu nude mice (5 mice/group) using a trocar needle. At the time when the tumor volume reached 100 to 200 mm³, the administration was started. Am80 was orally administered once a day at a dose of 1 mg/kg for 21 continuous days, and DXL was intravenously administered at a dose of 3.5 mg/kg from Day 1, 3 times at intervals of 4 days. In the combination group, the agents were administered at the same doses as, and by the same administration methods as, those in the cases of administration of each single agent. Measurement of the tumor diameter was carried out until the next day of the completion of the administration (Day 22). The longitudinal diameter and the transverse diameter of the tumor were measured using a caliper, and the tumor volume (mm³) was determined according to the following formula: longitudinal diameter × transverse diameter² /2. In the control group, physiological saline was intraperitoneally administered from Day 1, 3 times at intervals of 4 days. As a result, the combination of Am80 and DXL showed a synergistic inhibitory activity on the cell growth of the slow-growing human breast cancer cell line MCF-7, which inhibitory activity was higher than the effect of each single agent. These results are shown in following Table 2.

**[Table 2]**

| | Day 1 | Day 5 | Day 9 | Day 13 | Day 17 | Day 22 |
|---|---|---|---|---|---|---|
| Control group | 150.1 | 155.7 | 205.5 | 253.8 | 276.7 | 320.3 |
| Am80 group | 155.3 | 151.3 | 155.5 | 208.9 | 229.4 | 261.4 |
| DXL group | 143.5 | 121.5 | 110.0 | 83.3 | 66.5 | 71.3 |
| Combination group | 141.2 | 131.6 | 100.1 | 61.3 | 50.5 | 45.1 |

### [Example 21]

### <Influence of Combination of Am80 and DNA Methylation Inhibitor 5-AZ on Growth Inhibition of Human Prostate Cancer Cell Line LNCaP>

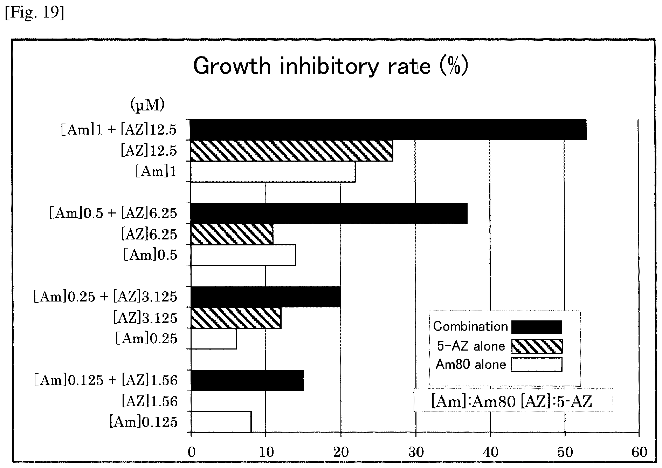

A human prostate cancer cell line LNCaP, containing cells positive for cancer stem cell markers, was cultured in RPMI1640 medium supplemented with 10% FBS, and the growth inhibitory action by the combination of Am80 and 5-AZ was studied. The IC50 values of the agents determined by 96 hours of the exposure were 2 µM and 25 µM, respectively. Thus, in order to study the growth inhibitory action, Am80 was used within the concentration range of 0.125 µM to 1 µM with a common ratio of 2, and 5-AZ was used within the concentration range of 1.56 µM to 12.5 µM with a common ratio of 2. In cases of use of these agents in combination, the growth inhibitory action was studied using these concentrations. The combination of Am80 and 5-AZ showed a synergistic effect on the growth inhibitory action (Fig. 19).

### [Example 22]

### <Influence of Combination of Am80 and Docetaxel (DXL) on Growth Inhibition of Human Prostate Cancer Cell Line LNCaP/Nude Mice>

A tumor piece of a human prostate cancer cell line LNCaP containing cells positive for cancer stem cell markers was subcutaneously transplanted to the dorsal part of each of 6-week-old female BALB/cAJcl-nu nude mice (4 mice/group) using a trocar needle. At the time when the tumor volume reached 100 to 200 mm³, the administration was started. Am80 was orally administered once a day at a dose of 1 mg/kg for 21 continuous days, and DXL was intravenously administered at a dose of 5 mg/kg from Day 1, 3 times at intervals of 4 days. In the combination group, the agents were administered at the same doses as, and by the same administration methods as, those in the cases of administration of each single agent. Measurement of the tumor diameter was carried out until the next day of the completion of the administration (Day 22). The longitudinal diameter and the transverse diameter of the tumor were measured using a caliper, and the tumor volume (mm³) was determined according to the following formula: longitudinal diameter × transverse diameter² /2. In the control group, physiological saline was intraperitoneally administered from Day 1, 3 times at intervals of 4 days. As a result, the combination of Am80 and DXL showed a synergistic inhibitory activity on the cell growth of the human prostate cancer cell line LNCaP, which inhibitory activity was higher than the effect of each single agent. These results are shown in following Table 3.

**[Table 3]**

| | Day 1 | Day 5 | Day 9 | Day 13 | Day 17 | Day 22 |
|---|---|---|---|---|---|---|
| Control group | 120.8 | 162.7 | 225.5 | 317.3 | 478.7 | 612.4 |
| Am80 group | 121.3 | 162.1 | 205.8 | 282.8 | 409.9 | 436.3 |
| DXL group | 126.7 | 111.3 | 90.7 | 73.7 | 72.5 | 70.2 |
| Combination group | 130.1 | 111.4 | 90.3 | 61.6 | 60.3 | 47.7 |

It could be confirmed that use of the retinoid agonist tamibarotene (Am-80) alone or in combination with the rexinoid agonist bexarotene (Targretin) suppresses expression of markers of human pancreatic cancer stem cells and the growth of those cells, and that its/their use in combination with various anticancer drugs enhances the effects the anticancer drugs. It could also be confirmed that use of Am80 in combination with various anticancer drugs enhances the effects of the anticancer drugs on various cancers.

## Claims

1. A composition comprising tamibarotene (Am-80) and a further anticancer drug for use in reducing cancer stem cells in a subject.

2. The composition for use according to claim 1, wherein tamibarotene (Am-80) is contained in the composition in an amount of 0.5 to 20 mg; and said anticancer drug is contained in the composition in an amount of 1.0 to 1000 mg.

3. The composition for use according to claim 1 or 2, wherein said anticancer drug is selected from the group consisting of DNA-interacting agents, antimetabolites, tubulin-interacting agents, celecoxib, rofecoxib, erlotinib, gefitinib, imatinib, azacytidine, valproic acid, SAHA, Romidepsin, and hormones.

4. The composition for use according to claim 3, wherein said DNA-interacting agent is selected from the group consisting of cyclophosphamide, melphalan, cisplatin, carboplatin, camptothecin, etoposide, daunorubicin, and doxorubicin.

5. The composition for use according to claim 3, wherein said antimetabolite is selected from the group consisting of methotrexate, trimethotrexate, 5-fluorouracil, 5-aza-dC, azacytidine, cytarabine, gemcitabine and fludarabine.

6. The composition for use according to claim 3, wherein said tubulin-interacting agent is selected from the group consisting of paclitaxel and docetaxel.

7. The composition for use according to claim 3, wherein said hormone is selected from the group consisting of leuprolide acetate, goserelin acetate, tamoxifen, flutamide, bicalutamide and enzalutamide.

8. The composition for use according to claim 3, wherein said anticancer drug comprises a combination of:
docetaxel and oxaliplatin; or
docetaxel and celecoxib, rofecoxib, erlotinib, gefitinib or imatinib.

9. The composition for use according to any one of claims 1 to 8, wherein said cancer of the cancer stem cells is selected from the group consisting of acute myeloid leukemia, non-Hodgkin's lymphoma, pancreatic cancer, hepatoma, colon cancer, lung cancer, breast cancer, prostate cancer and ovarian cancer.

10. The composition for use according claim 9, wherein said cancer of the cancer stem cells is acute myeloid leukemia or breast cancer.

## Patentansprüche

1. Zusammensetzung, umfassend Tamibaroten (Am-80) und ein weiteres Antikrebsmittel zur Verwendung bei der Verminderung von Krebsstammzellen in einem Patienten.

2. Zusammensetzung zur Verwendung nach Anspruch 1, wobei Tamibaroten (Am-80) in der Zusammensetzung in einer Menge von 0,5 bis 20 mg enthalten ist; und das Antikrebsmittel in der Zusammensetzung in einer Menge von 1,0 bis 1000 mg enthalten ist.

3. Zusammensetzung zur Verwendung nach Anspruch 1 oder 2, wobei das Antikrebsmittel ausgewählt ist aus der Gruppe, bestehend aus DNA-interagierenden Mitteln, Antimetaboliten, Tubulin-interagierenden Mitteln, Celecoxib, Rofecoxib, Erlotinib, Gefitinib, Imatinib, Azacytidin, Valproinsäure, SAHA, Romidepsin und Hormonen.

4. Zusammensetzung zur Verwendung nach Anspruch 3, wobei das DNAinteragierende Mittel ausgewählt ist aus der Gruppe, bestehend aus Cyclophosphamid, Melphalan, Cisplatin, Carboplatin, Camptothecin, Etoposid, Daunorubicin und Doxorubicin.

5. Zusammensetzung zur Verwendung nach Anspruch 3, wobei der Antimetabolit ausgewählt ist aus der Gruppe, bestehend aus Methotrexat, Trimethotrexat, 5-Fluoruracil, 5-Aza-dC, Azacytidin, Cytarabin, Gemcitabin und Fludarabin.

6. Zusammensetzung zur Verwendung nach Anspruch 3, wobei das Tubulininteragierende Mittel ausgewählt ist aus der Gruppe, bestehend aus Paclitaxel und Docetaxel.

7. Zusammensetzung zur Verwendung nach Anspruch 3, wobei das Hormon ausgewählt ist aus der Gruppe, bestehend aus Leuprolidacetat, Goserelinacetat, Tamoxifen, Flutamid, Bicalutamid und Enzalutamid.

8. Zusammensetzung zur Verwendung nach Anspruch 3, wobei das Antikrebsmittel eine Kombination aus:
Docetaxel und Oxaliplatin; oder
Docetaxel und Celecoxib, Rofecoxib, Erlotinib, Gefitinib oder Imatinib umfasst.

9. Zusammensetzung zur Verwendung nach einem der Ansprüche 1 bis 8, wobei der Krebs der Krebsstammzellen ausgewählt ist aus der Gruppe, bestehend aus akuter myeloischer Leukämie, Non-Hodgkin-Lymphom, Bauchspeicheldrüsenkrebs, Hepatom, Dickdarmkrebs, Lungenkrebs, Brustkrebs, Prostatakrebs und Eierstockkrebs.

10. Zusammensetzung zur Verwendung nach Anspruch 9, wobei der Krebs der Krebsstammzellen akute myeloische Leukämie oder Brustkrebs ist.

## Revendications

1. Composition comprenant du tamibarotène (Am-80) et un médicament anticancéreux supplémentaire pour utilisation dans la réduction des cellules souches cancéreuses chez un sujet.

2. Composition pour utilisation selon la revendication 1, dans laquelle le tamibarotène (Am-80) est contenu dans la composition en une quantité de 0,5 à 20 mg ; et ledit médicament anticancéreux est contenu dans la composition en une quantité de 1,0 à 1000 mg.

3. Composition pour utilisation selon la revendication 1 ou 2, dans laquelle ledit médicament anticancéreux est sélectionné dans le groupe constitué par: des agents interagissant avec l'ADN, des antimétabolites, dans agents interagissant avec la tubuline, le célécoxib, le rofécoxib, l'erlotinib, le gefitinib, l'imatinib, l'azacitidine, l'acide valproïque, le SAHA, le romidepsin et des hormones.

4. Composition pour utilisation selon la revendication 3, dans laquelle ledit agent interagissant avec l'ADN est sélectionné dans le groupe constitué par: le cyclophosphamide, le melphalan, le cisplatine, le carboplatine, la camptothécine, l'étoposide, la daunorubicine et la doxorubicine.

5. Composition pour utilisation selon la revendication 3, dans laquelle ledit antimétabolite est sélectionné dans le groupe constitué par: le méthotrexate, le triméthotrexate, le 5-fluorouracile, la 5-aza-dC, l'azacitidine, la cytarabine, la gemcitabine et la fludarabine.

6. Composition pour utilisation selon la revendication 3, dans laquelle ledit agent interagissant avec la tubuline est sélectionné dans le groupe constitué par: le paclitaxel et le docétaxel.

7. Composition pour utilisation selon la revendication 3, dans laquelle ladite hormone est sélectionnée dans le groupe constitué par: l'acétate de leuprolide, l'acétate de goséréline, le tamoxifène, le flutamide, le bicalutamide et l'enzalutamide.

8. Composition pour utilisation selon la revendication 3, dans laquelle ledit médicament anticancéreux comprend une combinaison de :
docétaxel et oxaliplatine ; ou
docétaxel et célécoxib, rofécoxib, erlotinib, gefitinib ou imatinib.

9. Composition pour utilisation selon l'une quelconque des revendications 1 à 8, dans laquelle ledit cancer des cellules souches cancéreuses est sélectionné dans le groupe constitué par une leucémie myéloïde aiguë, un lymphome non hodgkinien, un cancer du pancréas, un hépatome, un cancer du côlon, un cancer du poumon, un cancer du sein, un cancer de la prostate et un cancer ovarien.

10. Composition pour utilisation selon la revendication 9, dans laquelle ledit cancer des cellules souches cancéreuses est une leucémie myéloïde aiguë ou un cancer du sein.
